# EUROPÄISCHE PATENTSCHRIFT

(11) **EP 1 084 091 B1**
(45) Veröffentlichungstag und Bekanntmachung des Hinweises auf die Patenterteilung: **23.07.2003**
(21) Anmeldenummer: 99925022.8
(22) Anmeldetag: 21.05.1999
(51) Int. Cl.: C07C 17/16, C07C 41/22, C07B 39/00, B01J 31/02

(54) **VERFAHREN ZUR HERSTELLUNG VON ALKYL-, ALKENYL- UND ALKINYLCHLORIDEN**
METHOD FOR PRODUCING ALKYL CHLORIDE, ALKENYL CHLORIDE AND ALKINYL CHLORIDE
PROCEDE POUR PREPARER DES CHLORURES D'ALKYLE, D'ALCENYLE ET D'ALCYNYLE

(30) Priorität: 04.06.1998 DE 19824929
(43) Veröffentlichungstag der Anmeldung: 21.03.2001
(73) Patentinhaber: BASF AKTIENGESELLSCHAFT, 67056 Ludwigshafen (DE)
(72) Erfinder: WEYER, Hans-Jürgen, D-67240 Bobenheim-Roxheim (DE); STAMM, Armin, D-55128 Mainz (DE); WEBER, Theodor, D-67063 Ludwigshafen (DE); HENKELMANN, Jochem, D-68165 Mannheim (DE)
(86) Internationale Anmeldenummer: EP9903489
(87) Internationale Veröffentlichungsnummer: WO99062848

(56) Entgegenhaltungen:
- EP-A- 0 514 683
- EP-A- 0 645 357
- DE-A- 19 737 619
- US-A- 3 927 106
- US-A- 4 400 329
- KAMIJO T ET AL: "A novel, one-step conversion of alcohols into alkyl bromides or iodides" CHEM. PHARM. BULL. (CPBTAL,00092363);1983; VOL.31 (11); PP.4189-92, XP002111415 Kissei Pharm. Co., Ltd.;Cent. Res. Lab.; Matsumoto; 399-65; Japan (JP)

## Beschreibung

Die vorliegende Erfindung betrifft ein neues Verfahren zur Herstellung von Alkyl-, Alkenyl- und Alkinylchloriden durch Umsetzung von Alkoholen mit Chlorierungsmitteln in Gegenwart von Harnstoffverbindungen. Alkyl-, Alkenyl- und Alkylchloride sind bekannt und eignen sich als wertvolle Zwischenprodukte organischer Synthesen. Sie werden nach bekannten Verfahren durch Umsetzung von Alkoholen mit Phosgen in Gegenwart von Katalysatoren erhalten.

So ist aus der GB-A 2 182 039 ein Verfahren zur Herstellung von Alkylchloriden aus Alkoholen durch Umsetzung mit Phosgen in Gegenwart von Triarylphosphinoxiden oder Triarylphosphinsulfiden als Katalysatoren bekannt. In der EP-A 514 683 werden aliphatische Phosphorverbindungen als Katalysatoren vorgeschlagen.

Aus EP-A-200403 ist die Halogenierung von Alkoxyverbindungen in Gegenwart von unsubstituiertem Harnstoff bekannt.

Nachteilig an diesen Verfahren ist der Anfall phosphorhaltiger Destillationsrückstände, deren Entsorgung wegen der Bildung von Phosphorsäuren erschwert ist.

EP-A 0 645 357 offenbart die Herstellung von Alkylchloriden durch Umsetzung der entsprechenden Alkohole mit Phosgen oder Thionylchlorid in Gegenwart eines N,N-disubstituierten Formamids oder deren Hydrochloride.

Der Erfindung liegt die Aufgabe zugrunde, dem vorgenannten Nachteil abzuhelfen, insbesondere ein Verfahren zur Herstellung von Alkyl-, Alkenyl- und Alkinylchloriden, bereitzustellen, das mit geringen Katalysatormengen auskommt und die Bildung von Nebenprodukten weitgehend vermeidet.

Die Aufgabe wird gelöst durch ein Verfahren zur Herstellung von Alkyl-, Alkenyl- und Alkinylchloriden aus Alkoholen der Formel 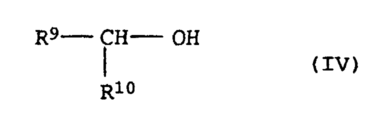 in der R⁹ und R¹⁰ Wasserstoff, Alkyl-, Alkenyl-, Alkinyl-, Alkoxy-, Aryloxy-, Hydroxyalkyl-, Hydroxyalkenyl- oder Hydroxyalkinylgruppen bezeichnen, durch Umsetzung mit einem Chlorierungsmittel in Gegenwart eines Katalysators, das dadurch gekennzeichnet ist, daß als Katalysator eine Harnstoffverbindung der Formel (I)

R¹R²N-CX-NR³R⁴ (I),

in der die Reste R¹, R², R³ und R⁴ gleich oder verschieden sein können und unabhängig voneinander gegebenenfalls mit C₁- bis C₄-Alkoxy, C₂- bis C₄-Acyl, C₂- bis C₄-Acyloxy, Phenoxy, C₂- bis C₈-Dialkylamino, Halogen, Nitro und/oder Cyano ein- bis dreifach substituiertes C₁- bis C₂₀-Alkyl, C₂- bis C₁₂-Alkenyl, C₂- bis C₁₂-Alkinyl oder gegebenenfalls mit C₁- bis C₄-Alkyl, C₁- bis C₄-Alkoxy, C₂- bis C₄-Acyl, C₂- bis C₄-Acyloxy, Phenoxy, C₂- bis C₈-Dialkylamino, Halogen, Nitro und/oder Cyano ein- bis dreifach substituiertes C₃- bis C₁₂-Cycloalkyl, C₄- bis C₁₂-Alkylcycloalkyl-, C₄- bis C₁₂-Cycloalkylalkyl, Heterocycloalkyl, C₅- bis C₂₀-Heterocycloalkyl-alkyl, C₆- bis C₁₄-Aryl, C₇- bis C₂₀-Arylalkyl oder C₇- bis C₀-Alkylaryl bedeutet haben oder in der einer der Reste R¹ oder R² gemeinsam mit einem der Reste R³ oder R⁴ eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und Cyano einbis dreifach substituierte C₂-C₁₂-Alkylenkette, die durch eine Ether-, Thioether-, tertiäre Amino-, Keto-, Lacton-, N-alkylsubstituierte Lactam- oder Sulfongruppe unterbrochen sein kann, bedeutet und in der X für ein Sauerstoff oder Schwefelatom steht und/oder einer Harnstoffverbindung der allgemeinen Formel II 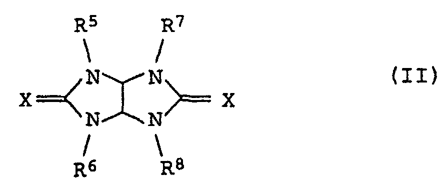 worin X die angegebene Bedeutung hat R⁵, R⁶, R⁷ und R⁸ gleich oder verschieden sein können und unabhängig voneinander die für R¹ bis R⁴ gegebene Bedeutung haben
und/oder
einer Verbindung der allgemeinen Formel (III), 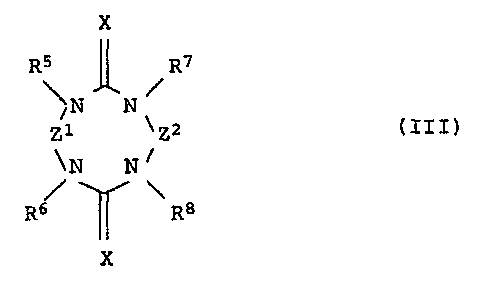 worin X, R⁵ bis R⁸ die oben angegebenen Bedeutungen und Z¹, Z², die gleich oder verschieden sein können, eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und/oder Cyano ein- bis dreifach substituierte Methylen-, Ethylen- oder Vinylengruppe bedeuten, eingesetzt wird.

Die organischen Substituenten R¹ bis R⁸, in den Verbindungen I, II und III haben unabhängig voneinander folgende Bedeutungen:
- C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl, insbesondere C₁- bis C₄-Alkyl wie Methyl, Ethyl, n-Propyl, iso-Propyl, n-Butyl, iso-Butyl, sec.-Butyl und tert.-Butyl,
- C₂-C₁₂-Alkenyl, bevorzugt C₂-C₈-Alkenyl, wie Vinyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-propenyl, 1-Ethyl-1-propenyl, 1-Ethyl-2-propenyl, 1-Hexenyl, 2-Hexenyl, 3-Hexenyl, 4-Hexenyl, 5-Hexenyl, besonders bevorzugt Vinyl, 2-Propenyl und 1-Butenyl,
- C₂- bis C₁₂-Alkinyl, bevorzugt C₂-C₈-Alkinyl, wie Ethinyl, 1-Propinyl, 2-Propinyl, 1-Butinyl, 3-Butinyl, 1-Methyl-2-propinyl, 1-Pentinyl, 2-Pentinyl, 3-Pentinyl, 1-Methyl-2-butinyl, 1-Methyl-3-butinyl,2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, besonders bevorzugt Ethinyl, 1-Propinyl und 1-Butinyl.

Die genannten Gruppen können mit C₁-C₄-Alkoxy, C₂-C₄-Acyl, C₂-C₄-Acyloxy, Phenoxy, C₂-C₈-Dialkylamino, Halogen, Nitro und/oder Cyano substituiert sein. Bevorzugt sind substituierte Alkylreste, besonders bevorzugt mit Halogen oder Cyano substituierte Alkylreste, wie Cyanmethyl, Chlormethyl.

R¹ bis R⁸ stehen weiterhin für:
- C₃- bis C₁₂-Cycloalkyl, bevorzugt C₅- bis C₈-Cycloalkyl wie Cyclopentyl, Cyclohexyl, Cycloheptyl und Cyclooctyl, besonders bevorzugt Cyclopentyl und Cyclohexyl,
- C₄- bis C₁₂-Alkylcycloalkyl, bevorzugt C₅- bis C₁₀-Alkylcycloalkyl, besonders bevorzugt C₅- bis C₈-Alkylcycloalkyl,
- C₄- bis C₁₂-Cycloalkyl-alkyl, bevorzugt C₅- bis C₁₀-Cycloalkyl-alkyl, besonders bevorzugt C₅- bis C₈-Cycloalkyl-alkyl,
- C₅- bis C₂₀-Alkyl-cycloalkyl-alkyl, bevorzugt C₆- bis C₁₆-Alkyl-cycloalkyl-alkyl, besonders bevorzugt C₇- bis C₁₂-Alkylcycloalkyl-alkyl,
- Heterocycloalkyl- wie ein 5- oder 6-gliedriger Ring oder einem oder zwei O-, N- und/oder S-Atomen im Ring, der aromatisch oder nichtaromatisch sein kann, wie 2- oder 3-Furyl, 2- oder 3-Thienyl, 2- oder 3-Pyrrolyl, 2- oder 4-Imidazolyl, 2- oder 3-Oxazolyl, 2- oder 3-Oxazolyl, 2-oder 3-Thiazolyl, Pyridinyl, Morpholyl, Thiomorpholyl und Pyrazolyl,
- C₆- bis C₁₄-Aryl wie Phenyl, 1-Naphthyl und 2-Naphthyl, bevorzugt Phenyl,
- C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
- C₇ bis C₂₀-Arylalkyl, bevorzugt C₇- bis C₁₆-Aralkyl, bevorzugt C₇- bis C₁₂-Phenalkyl wie Benzyl, 1-Phenylethyl, 2-Phenylethyl, 3-Phenylpropyl, 1-Phenylbutyl, 2-Phenylbutyl, 3-Phenylbutyl, 4-Phenylbutyl, besonders bevorzugt Benzyl, 1-Phenylethyl und 2-Phenylethyl.

Die genannten Gruppen können mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und/oder Cyano substituiert sein.

Als Harnstoffverbindungen der Formeln I, II oder III werden bevorzugt jene verwendet, die bei Reaktionsbedingungen flüssig sind, besonders bevorzugt N,N-Dimethylethylenharnstoff, N,N-Dimethylpropylenharnstoff, N,N,N',N'-Tetrabutylharnstoff und N,N,N',N'-Tetramethylthioharnstoff, N-Chlormethyl-N'-cyanomethylpropylenharnstoff, N-Methyl-N' -ethylpropylenharnstoff, 1,3-Dimethyl-1,3-dihydrobenzimidazol-2-on, 1-Methyl-3-phenylimidazolidin-2,4,5-trion, 1,3,4,6-Tetramethyl-1,3,4,6-tetrahydroimidazo[4,5-D]imidazol-2,5-dion und 4-Methoxy-1-methyl-3-phenylimidazolidin-2-thion.

Die genannten Harnstoffverbindungen können als solche, in Form ihrer Salze mit Halogenwasserstoffsäuren, beispielsweise als Hydrochloride, oder in Form ihrer durch Umsetzung mit Phosgen erhältlichen Salze (Vilsmeier Salze) eingesetzt werden.

Unter den definitionsgemäßen Alkoholen der Formel (IV) werden im Hinblick auf die gewünschten Verfahrensprodukte solche bevorzugt, in denen die Reste R⁹ und R¹⁰ folgende Bedeutung haben:
- Wasserstoff,
- C₁-C₂₂-Alkyl, vorzugsweise C₄-C₁₈-Alkyl, darunter vorzugsweise C₆-C₁₀-Alkyl wie vor allem n-Hexyl und n-Octyl;
- C₃-C₂₂-Alkenyl, vorzugsweise C₄-C₆-Alkenyl wie insbesondere Butenyl;
- C₃-C₂₂-Alkinyl, vorzugsweise C₃-C₈-Alkinyl wie vor allem Propinyl;
- C₁-C₂₂-Alkoxy, vorzugsweise C₁-C₄-Alkoxy wie insbesondere Propoxy;
- ein- oder zweikernige Aryloxy wie vorzugsweise Phenyloxy, wobei die aromatischen Ringe Heteroatome wie Sauerstoff, Schwefel oder Stickstoff enthalten können und/oder bis zu drei C₁-C₁₂-Alkylgruppen, Halogen wie Fluor, Chlor und Brom oder C₁-C₄-Alkoxygruppen als Substituenten tragen können;
- C₁-C₁₆-Hydroxyalkyl, vorzugsweise C₂-C₁₂-Hydroxyalkyl, darunter vorzugsweise C₄-C₈-Hydroxyalkyl wie vor allem Hydroxybutyl, Hydroxyhexyl und Hydroxyoctyl;
- C₁-C₁₆-Hydroxyalkenyl, vorzugsweise C₂-C₁₂-Hydroxyalkenyl, darunter vorzugsweise C₄-C₈-Hydroxyalkenyl wie insbesondere Hydroxybutenyl, Hydroxyhexenyl und Hydroxyoctenyl;
- C₁-C₁₆-Hydroxyalkinyl, vorzugsweise C₂-C₁₂-Hydroxyalkinyl, darunter vorzugsweise C₄-C₈-Hydroxyalkinyl wie vor allem Hydroxybutinyl, Hydroxyhexinyl und Hydroxyoctinyl.

Hierbei können die Reste außer Wasserstoff ihrerseits Substituenten tragen, vorzugsweise C₁-C₄-Alkyl, C₁-C₄-Ester, Cyano, Halogen wie vor allem Fluor, Chlor und Brom, Aryl wie insbesondere Phenyl und 4-Methoxyphenyl und Aryloxy, wie vorzugsweise Phenyloxy.

Bevorzugte Alkohole sind:
- 2- (4-Methoxyphenyl)ethanol-1
- 2-Ethylhexanol-1
- n-Octanol-1
- But-3-en-ol-1
- Propinol
- Butandiol-1,4
- Octandiol-1,8,
im Hinblick auf die gewünschten Verfahrensprodukte besonders bevorzugt ist Hexandiol-1,6.

Als Chlorierungsmittel können an sich bekannte Reagenzien wie Phosgen, Thionylchlorid und Oxalylchlorid eingesetzt werden, wobei die Verwendung von Phosgen bevorzugt ist.

Häufig ist die zusätzliche Mitverwendung von Chlorwasserstoff vorteilhaft, und zwar in Mengen von 5 bis 100 mol-%, vorzugsweise 20 bis 40 mol-%, des Alkohols.

Das erfindungsgemäße Verfahren kann als homogen katalysate Flüssigphasenreaktion durchgeführt werden. Als flüssiges Reaktionsmedium kommen der einzusetzende primäre, sekundäre oder tertiäre Alkohol oder ein inertes Lösungsmittel oder deren Mischungen in Frage. Als inertes Lösungsmittel eignen sich aromatische Kohlenwasserstoffe wie Toluol, Xylol oder Benzol, halogenierte Kohlenwasserstoffe wie Trichlorethan, Chlorbenzol oder Dichlorbenzol oder Ester wie Ethylacetat oder Butylacetat.

Das erfindungsgemäße Verfahren kann kontinuierlich oder diskontinuierlich durchgeführt werden. Bevorzugt wird das erfindungsgemäße Verfahren kontinuierlich geführt, beispielsweise in einem Rührkessel, in einer Rührkesselkaskade, in einem Schlaufenreaktor oder in einer Gegenstromkolonne. Die Umsetzungstemperatur beträgt im allgemeinen -20 bis 180°C, bevorzugt 0 bis 120°C, besonders bevorzugt 40 bis 100°C. Die Umsetzung wird im allgemeinen bei Drücken von 0,01 bis 50 bar, bevorzugt 0,5 bis 5 bar, besonders bevorzugt bei Atmosphärendruck durchgeführt.

Die Harnstoffverbindung I, II und/oder III wird im allgemeinen, bezogen auf die Menge des eingesetzten Alkohols (IV), in Mengen von 0,01 bis 20 Mol-% eingesetzt. Die Menge des Katalysators richtet sich auch danach, ob die Umsetzung in dem eingesetzten Alkohol allein oder in Gegenwart eines Lösungsmittels durchgeführt wird.

Die verwendete Menge der Harnstoffverbindung I, II und/oder III liegt bevorzugt zwischen 0,001 und 1 Mol-%, besonders bevorzugt zwischen 0,0002 bis 01 Mol-%, besonders bevorzugt zwischen 0,005 und 0,05 Mol-%.

Das Molverhältnis des Chlorierungsmittels zum Alkohol (IV) beträgt im allgemeinen 0,5:1 bis 50:1. Üblicherweise wird man mit einem Überschuß an Chlorierungsmittel arbeiten, da andernfalls unumgesetzter Alkohol zurückbleibt. Das Molverhältnis des Chlorierungsmittels zum Alkohol (IV) beträgt bevorzugt 1:1 bis 2:1, besonders bevorzugt 1:1 bis 1,5:1, insbesondere 1:1 bis 1,2:1.

Der Chlorierungsreaktion können sich ein oder mehrere Schritte zur Aufreinigung des Reaktionsproduktes anschließen. So kann gegebenenfalls der flüssige Reaktionsaustrag der Phosgenierungsreaktion durch eine mechanische Abtrennung wie eine Klärfiltration von unlöslichen Verunreinigungen befreit werden. Oft ist ein solcher mechanischer Abtrennschritt schon ausreichend, um ein Produkt ausreichend hoher Reinheit zu erhalten, und eine weitere Aufarbeitung kann entfallen. Es können sich jedoch weitere Aufreinigungsschritte zur Abtrennung löslicher Verunreinigungen, beispielsweise durch Destillation oder Umkristallisieren, anschließen.

Der katalysatorhaltige Destillationsrückstand kann in die Reaktion zurückgeführt werden.

Die nach dem erfindungsgemäßen Verfahren auf wirtschaftliche Weise erhältlichen Alkyl-, Alkenyl- und Alkinylchloride sind bekanntermaßen wertvolle Zwischenprodukte für organische Synthesen, insbesondere von Pflanzenschutzmitteln, Galvanohilfsmitteln und Kunststoffvorprodukten.

Die Erfindung wird durch die nachstehenden Beispiele erläutert.

### Beispiele 1 bis 5

In eine Mischung aus 0,5 Mol des jeweiligen Alkylchlorids und 0,001 Mol-% der Harnstoffverbindung, bezogen auf den Alkohol, wurden bei einer Temperatur von 120-130°C im Laufe von 5 Stunden b mol Phosgen und 2 Mol des Alkohols (IV) zugesetzt. Anschließend wurde das Gemisch noch eine Stunde bei der Reaktionstemperatur gehalten. Danach wurde überschüssiges Phosgen mit Stickstoff aus dem Gemisch ausgetrieben und das so erhaltene Rohprodukt wurde nach einer Klärfiltration gaschromatographisch untersucht.

Einzelheiten zu diesen Versuchen sind der nachstehenden Tabelle 1 zu entnehmen.

### Beispiel 6

In eine Mischung aus 0,5 Mol 1,4-Dichlorbutan und 0,001 Mol-% N,N'-Dimethylpropylenharnstoff, bezogen auf 1,4-Butandiol, wurden bei einer Temperatur von 120-130°C im Laufe von 7 Stunden b mol Thionylchlorid und 2 Mol Butandiol-1,4 zugesetzt. Anschließend wurde das Gemisch noch eine Stunde bei der Reaktionstemperatur gehalten. Danach wurde überschüssiges Thionylchlorid mit Stickstoff aus dem Gemisch getrieben und das so erhaltene Rohprodukt wurde nach einer Klärfiltration gaschromatographisch untersucht.

Einzelheiten des Versuchs sind der nachstehenden Tabelle 1 zu entnehmen.

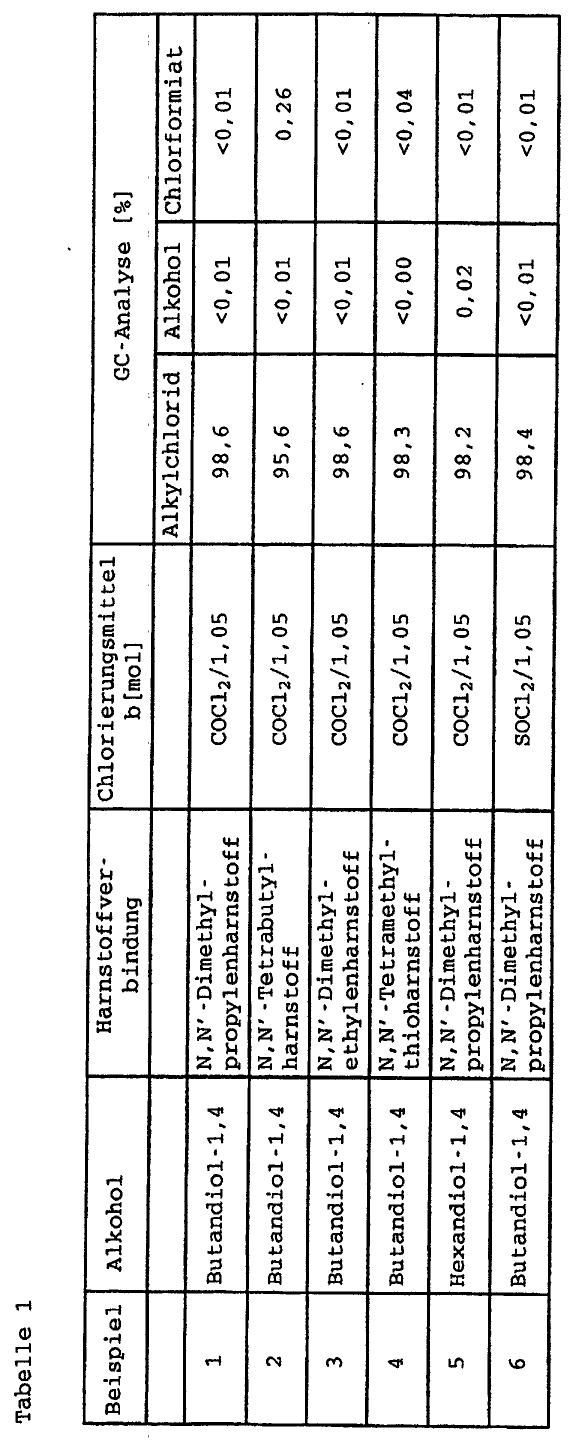

## Patentansprüche

1. Verfahren zur Herstellung von gegebenenfalls substituierten Alkyl-, Alkenyl- und Alkinylchloriden aus Alkoholen der Formel IV 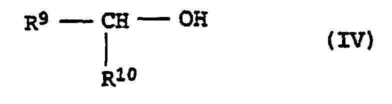 in der R⁹ und R¹⁰ Wasserstoff, Alkyl-, Alkenyl-, Alkinyl-, Alkoxy-, Aryloxy-, Hydroxyalkyl-, Hydroxyalkenyl- oder Hydroxyalkinylgruppen bezeichnen, durch Umsetzung mit einem Chlorierungsmittel in Gegenwart eines Katalysators, **dadurch gekennzeichnet, daß** man als katalysator eine Harnstoffverbindung der Formel (I)
R¹R²N-CX-NR³R⁴ (I),
in der die Reste R¹, R², R³ und R⁴ gleich oder verschieden sein können und unabhängig voneinander gegebenenfalls mit C₁bis C₄-Alkoxy, C₂- bis C₄-Acyl, C₂- bis C₄-Acyloxy, Phenoxy, C₂- bis C₈-Dialkylamino, Halogen, Nitro und/oder Cyano einbis dreifach substituiertes C₁- bis C₂₀-Alkyl, C₂- bis C₁₂-Alkenyl, C₂- bis C₁₂-Alkinyl oder gegebenenfalls mit C₁bis C₄-Alkyl, C₁- bis C₄-Alkoxy, C₂- bis C₄-Acyl, C₂- bis C₄-Acyloxy, C₂- bis C₈-Dialkylamino, Halogen, Nitro und/oder Cyano ein- bis dreifach substituiertes C₃- bis C₁₂-Cycloalkyl, C₄- bis C₁₂-Alkylcycloalkyl-, C₄- bis C₁₂-Cycloalkylalkyl, Heterocycloalkyl, C₅- bis C₂₀-Heterocycloalkyl-alkyl, C₆- bis C₁₄-Aryl, C₇- bis C₂₀-Arylalkyl oder C₇- bis C₂₀-Alkylaryl bedeuten oder in der einer der Reste R¹ oder R² gemeinsam mit einem der Reste R³ oder R⁴ eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und Cyano ein- bis dreifach substituierte C₂-C₁₂-Alkylenkette, die durch eine Ether-, Thioether-, tertiäre Amino-, Keto-, Lacton-, N-alkylsubstituierte Lactam- oder Sulfongruppe unterbrochen sein kann, bedeuten kann und in der X für ein Sauerstoff- oder Schwefelatom steht und/oder einer Harnstoffverbindung der allgemeinen Formel II 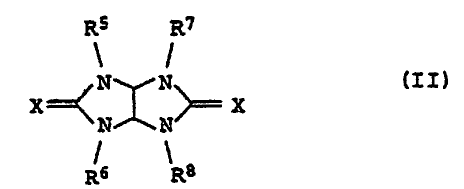 worin X die angegebene Bedeutung hat R⁵, R⁶, R⁷ und R⁸ gleich oder verschieden sein können und unabhängig voneinander die für R¹ bis R⁴ gegebene Bedeutung haben
und/oder
einer Verbindung der allgemeinen Formel (III), 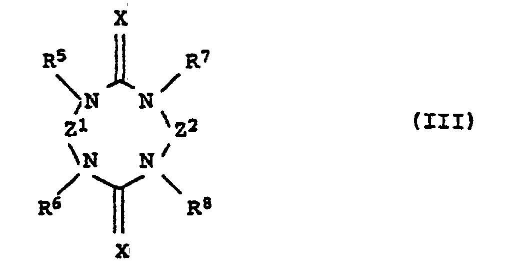 worin X, R⁵ bis R⁸ die oben angegebenen Bedeutungen und Z¹, Z², die gleich oder verschieden sein können, eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und/oder Cyano ein- bis dreifach substituierte Methylen-, Ethylen- oder Vinylengruppe bedeuten, einsetzt.

2. Verfahren nach Anspruch 1, **dadurch gekennzeichnet, daß** man als Katalysator N,N'-Dimethylethylenharnstoff, N,N'-Dimethylpropylenharnstoff, N,N,N',N'-Tetrabutylharnstoff und N,N,N',N'-Tetramethylthioharnstoff, N-Chlormethyl-N'-cyanomethylpropylenharnstoff, N-Methyl-N'-ethylpropylenharnstoff, 1,3-Dimethyl-1,3-dihydrobenzimidazol-2-on, 1-Methyl-3-phenylimidazolidin-2,4,5-trion, 1,3,4,6-Tetramethyl-1,3,4,6-tetrahydro-imidazo[4,5-D]imidazol-2,5-dion und 4-Methoxy-1-methyl-3-phenylimidazolidin-2-thion verwendet.

3. Verfahren nach einem der Ansprüche 1 bis 2, **dadurch gekennzeichnet, daß** man 0,0001 bis 1 Mol-% der Harnstoffverbindung, bezogen auf den Alkohol einsetzt.

4. Verfahren nach einem der Ansprüche 1 bis 3, **dadurch gekennzeichnet, daß** man die Umsetzung bei Temperaturen von -40 bis 100°C und Atomosphärendruck durchführt.

5. Verfahren nach einem der Ansprüche 1 bis 4, **dadurch gekennzeichnet, daß** man die Umsetzung in Gegenwart eines Lösungsmittels durchführt.

## Claims

1. A process for preparing unsubstituted or substituted alkyl, alkenyl and alkynyl chlorides from alcohols of the formula IV 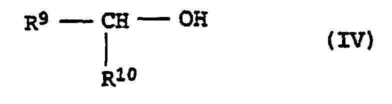 in which R⁹ and R¹⁰ are hydrogen, alkyl, alkenyl, alkynyl, alkoxy, aryloxy, hydroxyalkyl, hydroxyalkenyl or hydroxyalkynyl groups, by reaction with a chlorinating agent in the presence of a catalyst, wherein the catalyst is a urea compound of the formula (I)
R¹R²N-CX-NR³R⁴ (I),
in which the radicals R¹, R², R³ and R⁴ can be identical or different and are, independently of one another, optionally mono- to tri-C₁-C₄-alkoxy-, C₂-C₄-acyl-, C₂-C₄-acyloxy-, phenoxy-, C₂-C₈-dialkylamino-, halo-, nitro- and/or cyano-substituted C₁-C₂₀-alkyl, C₂-C₁₂-alkenyl, C₂-C₁₂-alkynyl or optionally mono- to tri-C₁-C₄-alkyl-, C₁-C₄-alkoxy-, C₂-C₄-acyl-, C₂-C₄-acyloxy-, C₂-C₈-dialkylamino-, halo-, nitro- and/or cyano-substituted C₃-C₁₂-cycloalkyl, C₄-C₁₂-alkylcycloalkyl, C₄-C₁₂-cycloalkylalkyl, heterocycloalkyl, C₅-C₂₀-heterocycloalkylalkyl, C₆-C₁₄-aryl, C₇-C₂₀-arylalkyl or C₇-C₂₀-alkylaryl, or in which one of the radicals R¹ or R² can be, together with one of the radicals R³ or R⁴, an optionally mono- to tri-C₁-C₄-alkyl-, C₁-C₄-alkoxy-, C₂-C₄-acyl-, C₂-C₄-acyloxy-, phenoxy-, C₂-C₈-dialkylamino-, halo-, nitro- and cyano-substituted C₂-C₁₂-alkylene chain which may be interrupted by an ether, thioether, tertiary amino, keto, lactone, N-alkyl-substituted lactam or sulfone moiety, and in which X is an oxygen or sulfur atom, and/or a urea compound of the general formula II 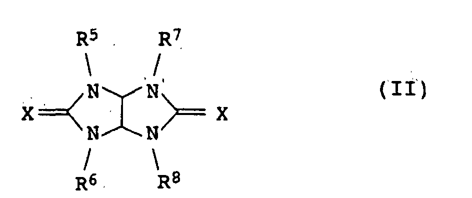 in which X has the stated meaning, and R⁵, R⁶, R⁷ and R⁸ can be identical or different and, independently of one another, have the meaning given for R¹ to R⁴
and/or
a compound of the general formula (III), 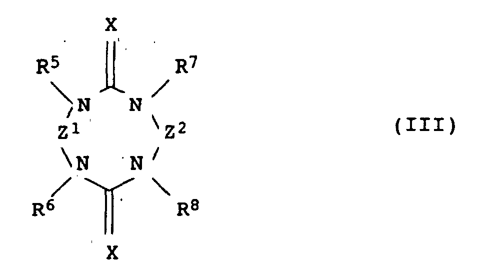 in which X, R⁵ to R⁸ have the abovementioned meanings and Z¹, Z², which may be identical or different, are an optionally mono- to tri-C₁-C₄-alkyl-, C₁-C₄-alkoxy-, C₂-C₄-acyl-, C₂-C₄-acyloxy-, phenoxy-, C₂-C₈-dialkylamino-, halo-, nitro- and/or cyano-substituted methylene, ethylene or vinylene group.

2. A process as claimed in claim 1, wherein the catalyst is N,N'-dimethylethyleneurea, N,N'-dimethylpropyleneurea, N,N,N',N'-tetrabutylurea and N,N,N',N'-tetramethylthiourea, N-chloromethyl-N'-cyanomethylpropyleneurea, N-methyl-N'-ethylpropyleneurea, 1,3-dimethyl-1,3-dihydrobenzimidazol- 2-one, 1-methyl-3-phenylimidazolidine-2,4,5-trione, 1,3,4,6-tetramethyl-1,3,4,6-tetrahydroimidazo[4,5-D]imidazole-2,5-dione and 4-methoxy-1-methyl-3-phenylimidazolidine-2-thione.

3. A process as claimed in either of claims 1 and 2, wherein from 0.0001 to 1 mol% of the urea compound is employed, based on the alcohol.

4. A process as claimed in any of claims 1 to 3, wherein the reaction is carried out at temperatures from -40 to 100°C and under atmospheric pressure.

5. A process as claimed in any of claims 1 to 4, wherein the reaction is carried out in the presence of a solvent.

## Revendications

1. Procédé pour la préparation des chlorures d'alkyle, d'alcényle et d'alcynyle éventuellement substitués, à partir d'alcools de formule IV 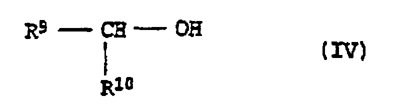 dans laquelle R⁹ et R¹⁰ représentent un atome d'hydrogène, un groupe alkyle, alcényle, alcynyle, alcoxy, aryloxy, hydroxyalkyle, hydroxyalcényle ou hydroxyalcynyle, au moyen d'une réaction avec un agent de chloration, en présence d'un catalyseur, **caractérisé en ce que** l'on met en oeuvre, en tant que catalyseur, un composé urée de formule (I)
R¹R²N-CX-NR³R⁴ (I),
dans laquelle les groupes R¹, R², R³ et R⁴ peuvent être identiques ou différents et représentent, indépendamment les uns des autres, un groupe alkyle en C₁ à C₂₀, alcényle en C₂ à C₁₂, alcynyle en C₂ à C₁₂ qui sont éventuellement substitués, une à trois fois, par un groupe alcoxy en C₁ à C₄, acyle en C₂ à C₄, acyloxy en C₂ à C₄, phénoxy, dialkylamino en C₂ à C₈, un atome d'halogène, un groupe nitro et/ou cyano, ou un groupe cycloalkyle en C₃ à C₁₂, alkylcycloalkyle en C₄ à C₁₂, cycloalkylalkyle en C₄ à C₁₂, hétérocycloalkyle, hétérocycloalkyl-alkyle en C₅ à C₂₀, aryle en C₆ à C₁₄, arylalkyle en C₇ à C₂₀ ou alkylaryle en C₇ à C₂₀ qui sont éventuellement substitués, une à trois fois, par un groupe alkyle en C₁ à C₄, alcoxy en C₁ à C₄, acyle en C₂ à C₄, acyloxy en C₂ à C₄, dialkylamino en C₂ à C₈, un atome d'halogène, un groupe nitro et/ou cyano, ou bien dans laquelle l'un des groupes R¹ ou R², conjointement avec l'un des groupes R³ ou R⁴, représente une chaîne alkylène en C₂ à C₁₂ qui est éventuellement substituée, une à trois fois, par un groupe alkyle en C₁ à C₄, alcoxy en C₁ à C₄, acyle en C₂ à C₄, acyloxy en C₂ à C₄, phénoxy, dialkylamino en C₂ à C₈, un atome d'halogène, un groupe nitro et cyano, et qui est éventuellement interrompue par un groupe éther, thioéther, amino tertiaire, céto, lactone, sulfone ou lactame à substitution N-alkyle, et dans laquelle X représente un atome d'oxygène ou de soufre,
et/ou un composé urée de formule générale II 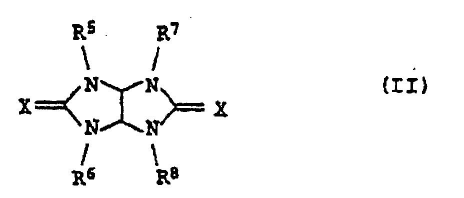 dans laquelle X prend la signification susmentionnée, et R⁵, R⁶, R⁷ et R⁸ peuvent être identiques ou différents et prennent, indépendamment les uns des autres, la signification susmentionnée pour les groupes R¹ à R⁴
et/ou
un composé de formule générale (III) 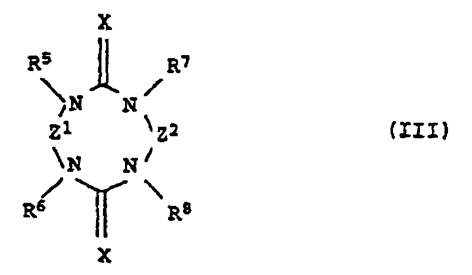 dans laquelle X et R⁵ à R⁸ prennent les significations susmentionnées, et Z¹ et Z², pouvant être identiques ou différents, représentent un groupe méthylène, éthylène ou vinylène qui sont éventuellement substitués, une à trois fois, par un groupe alkyle en C₁ à C₄, alcoxy en C₁ à C₄, acyle en C₂ à C₄, acyloxy en C₂ à C₄, phénoxy, dialkylamino en C₂ à C₈, un atome d'halogène, un groupe nitro et/ou cyano.

2. Procédé selon la revendication 1, **caractérisé en ce que** l'on met en oeuvre, en tant que catalyseur, la N,N'-diméthyléthylène-urée, la N,N'-diméthylpropylène-urée, la N,N,N',N'-tétrabutyl-urée et la N,N,N',N'-tétraméthylthio-urée, la N-chlorométhyl-N'-cyanométhylpropylène-urée, la N-méthyl-N'-éthylpropylène-urée, la 1,3-diméthyl-1,3-dihydrobenzimidazol-2-one, la 1-méthyl-3-phénylimidazolidin-2,4,5-trione, la 1,3,4,6-tétraméthyl-1,3,4,6-tétrahydro-imidazo[4, 5-D] imidazol-2, 5-dione et la 4-méthoxy-1-méthyl-3-phénylimidazolidin-2-thione.

3. Procédé selon l'une quelconque des revendications 1 à 2, **caractérisé en ce que** l'on met en oeuvre le composé urée à raison de 0,0001% à 1% en moles par rapport à l'alcool.

4. Procédé selon l'une quelconque des revendications 1 à 3, **caractérisé en ce que** l'on réalise la réaction à une température allant de -40°C à 100°C et sous la pression atmosphérique.

5. Procédé selon l'une quelconque des revendications 1 à 4, **caractérisé en ce que** l'on réalise la réaction en présence d'un solvant.
